(19) Europäisches Patentamt
European Patent Office
Office européen des brevets

(11) EP 0 845 467 B1

(12) **FASCICULE DE BREVET EUROPEEN**

(45) Date de publication et mention de la délivrance du brevet:
**13.10.1999 Bulletin 1999/41**

(51) Int Cl.6: **C07F 7/08**, C07F 7/21, A61K 7/42, A61K 7/48

(21) Numéro de dépôt: **97402639.5**

(22) Date de dépôt: **05.11.1997**

(54) **Nouveaux dérivés de filtres siliciés, compositions photoprotectrices les contenant et utilisations**

Silizium-enthaltende Sonnenschutzfilter, diese enthaltende Sonnenschutzzusammensetzungen und deren Verwendung

Silicon containing sunscreen agents, sunscreen composition containing them and their use

(84) Etats contractants désignés:
**AT BE CH DE ES FR GB IT LI NL SE**

(30) Priorité: **28.11.1996 FR 9614599**

(43) Date de publication de la demande:
**03.06.1998 Bulletin 1998/23**

(73) Titulaire: **L'OREAL**
**75008 Paris (FR)**

(72) Inventeurs:
- **Richard, Hervé**
  **93420 Villepinte (FR)**
- **Leduc, Madeleine**
  **29 rue des Boulets, 75011 Paris (FR)**

(74) Mandataire: **Miszputen, Laurent**
**L'OREAL-DPI**
**6 rue Bertrand Sincholle**
**92585 Clichy Cédex (FR)**

(56) Documents cités:
**EP-A- 0 305 059**
**EP-A- 0 358 584**
**EP-A- 0 392 882**
**WO-A-93/10745**
**US-A- 4 661 616**

- **ANDRIANOV, K.A. ET AL.: "reactions of 2,2-dimethyl-1-oxa-2-silacylohexane" ZHURNAL OBSHCHEI KHIMII, vol. 49, no. 10, 1979, pages 2250-2254, XP002032691**
- **CHEMICAL ABSTRACTS, vol. 108, no. 24, 13 juin 1988 Columbus, Ohio, US; abstract no. 205203p, CREED, D. ET AL.: "photochemical crosslinking of novel polycinnamate main-chain mesogens" XP002032692 & MOL. CRYST. LIQ. CRYST., 1988, pages 57-71,**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention concerne de nouveaux dérivés de cinnamamides, de benzalmalonamides et de benzalmalonates portant une chaîne siliconée courte sur la partie aromatique.

L'invention concerne également des compositions, notamment cosmétiques, contenant ces nouveaux dérivés, qui peuvent être destinées à la photoprotection de la peau et/ou des cheveux contre le rayonnement UV, en particulier le rayonnement solaire.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande croissante de moyens de contrôle de ce bronzage naturel afin de contrôler ainsi la couleur de leur peau. Il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreux composés destinés à la photoprotection (UV-A et/ou UV-B) de la peau ont été proposés à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne résistance à l'eau et à la transpiration (rémanence).

Parmi tous les composés aromatiques qui ont été préconisés à cet effet, on peut citer notamment les silicones à fonction cinnamate du document EP-A-305 059, les dérivés siliciés de benzylidène camphre, de benzophénone ou de benzotriazole décrits dans la demande WO93/10745, les dérivés de benzalmalonates siliciés décrits dans la demande EP-A-358 584 dans la demande EP-A-392 882 de la Demanderesse. Ces composés possèdent certes une bonne liposolubilité. Cependant, ces substances présentent des chaînes siliconées très longues et leur synthèse ainsi que leur incorporation dans les compositions cosmétiques, du fait de leur encombrement, sont fastidieuses. Enfin, leurs propriétés cosmétiques ne sont pas toujours satisfaisantes.

La présente invention vise à résoudre les problèmes ci-dessus en proposant de nouveaux dérivés de cinnamamides, de benzalmalonamides et de benzalmalonates portant une chaîne siliconée courte sur la partie aromatique qui présentent, outre une très bonne solubilité dans les corps gras, des propriétés cosmétiques améliorées. Plus précisément encore, il a été trouvé, selon la présente invention, qu'en greffant au moins un groupement cinnamamide, benzalmalonamide ou benzalmalonate sur une chaîne siliconée courte, en particulier sur une chaîne siliconée linéaire comprenant au maximum six atomes de Si, il était possible d'aboutir à de nouveaux composés obviant aux inconvénients des filtres de l'art antérieur, ces nouveaux composés présentant, outre des propriétés filtrantes très élevées, une très bonne solubilité dans les solvants organiques usuels et notamment les corps gras tels que les huiles, ainsi que d'excellentes propriétés cosmétiques, les rendant particulièrement appropriés à une utilisation comme filtres solaires dans des, ou pour la préparation de, compositions cosmétiques destinées à la protection de la peau et/ou des cheveux contre le rayonnement ultraviolet. Ces nouveaux composés, du fait de leur taille peu importante, sont de plus faciles à synthétiser.

La présente invention a ainsi pour premier objet de nouveaux composés qui sont caractérisés par le fait qu'ils répondent à l'une des formules (1) ou (2) suivantes :

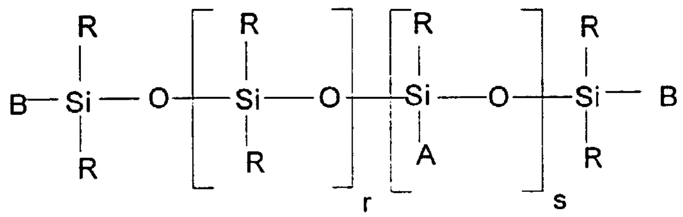

(1)

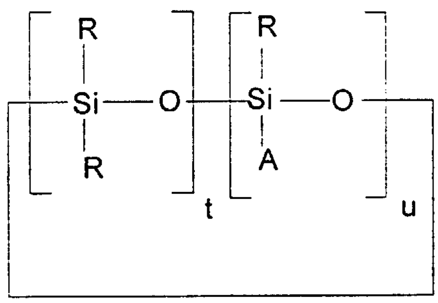

(2)

dans lesquelles :

- R, identiques ou différents sont choisis parmi les radicaux alkyles en $C_1$-$C_{10}$, linéaires ou ramifiés, saturés ou insaturés, le radical phényle et le radical trifluoro-3,3,3 propyle, au moins 80% en nombre des radicaux R étant le radical méthyle,
- B, identiques ou différents, sont choisis parmi les radicaux R et le radical A,
- r est un nombre entier choisi entre 0 et 3 inclusivement,
- s est 0 ou 1 et si s est 0, au moins un des deux symboles B est A,
- u est égal à 1 ou 2,
- t est un nombre entier entre 0 et 5 inclus,
- t + u est supérieur ou égal à 3,

et A est un radical de formule (3) suivante:

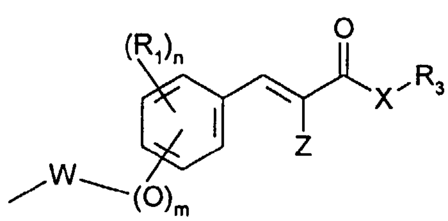

(3)

dans laquelle :

- $R_1$ représente un radical alcoxy en $C_1$-$C_{10}$ linéaire ou ramifié, un radical alkyle en $C_1$-$C_{10}$ linéaire ou ramifié, un radical alcényle en $C_2$-$C_8$ linéaire ou ramifié ou -$OSi(CH_3)_3$, deux $R_1$ adjacents pouvant former ensemble un groupement alkylidène dioxy dans lequel le groupe alkylidène contient de 1 à 2 atomes de carbone,
- n est 0, 1 ou 2,
- m est 0 ou 1,
- Z est l'hydrogène ou un radical -(C=O)$YR_3$,
- X et Y, identiques ou différents, représentent indépendamment l'oxygène ou un radical -$NR_2$-
- W est un radical alcane di-yle en $C_1$-$C_6$ linéaire ou ramifié, saturé ou insaturé, éventuellement substitué par un radical hydroxyle ou alcoyle en $C_2$-$C_8$, linéaire ou ramifié, saturé ou insaturé,
- $R_2$ est indépendamment hydrogène ou un radical alkyle en $C_1$-$C_8$,

- $R_3$ est indépendamment un radical alkyle en $C_1$-$C_{12}$ linéaire ou ramifié,
- $R_2$ et $R_3$, pris ensemble, pouvant former un hétérocycle,

avec la condition que lorsque Z = H, alors X est le radical $-NR_2-$.

Par hétérocycle, on entend de préférence un radical pipéridino, morpholino, pyrrolidino ou pipérazino, éventuellement substitué en position 4 par un radical alkyle en $C_1$-$C_6$.

Les composés de l'invention se différencient de ceux de la demande EP-A-392882 par le fait que la ou les chaînes siliconées greffées sur la ou les parties aromatiques des groupements cinnamamides, benzalmalonamides ou benzalmalonates possèdent au maximum six atomes de Si lorsqu'elles sont linéaires et au maximum sept atomes de Si lorsqu'elles sont cycliques. Ces composés, bien que possédant ainsi une chaîne siliconée courte, présentent une excellente liposolubilité et peuvent ainsi être utilisés à de grandes concentrations, ce qui confère aux compositions finales des indices de protection très élevés ; par ailleurs, ils se répartissent uniformément dans les supports cosmétiques classiques contenant au moins une phase grasse ou un solvant organique cosmétiquement acceptable et peuvent être ainsi appliqués sur la peau ou les cheveux pour constituer un film protecteur efficace. De plus, leurs propriétés cosmétiques sont très bonnes, à savoir en particulier que ces produits, par rapport aux silicones filtres de l'art antérieur, sont moins collantes et apportent plus de douceur.

En outre, les composés de l'invention présentent un excellent pouvoir filtrant intrinsèque à l'égard des rayonnements ultraviolet UV-A et UV-B.

Ces nouveaux dérivés de cinnamamides, de benzalmalonamides et de benzalmalonates siliciés peuvent ainsi être utilisés comme filtres solaires pour la peau humaine et les cheveux. Ils peuvent aussi être utilisés comme agents protecteurs de la lumière dans l'industrie des plastiques.

On préfère plus particulièrement les dérivés de cinnamamides, de benzalmalonates ou de benzalmalonamides bien définis de formules (1) ou (2) présentant au moins l'une des caractéristiques suivantes :

- R est méthyle,
- B est méthyle,
- r est 0 ou 1,
- s est 1,
- t + u est compris entre 3 et 5,
- n est 0 ou 1,
- $R_1$ est méthoxy,
- $R_3$ est méthyle ou éthyle,
- m est 1.

De préférence encore, les composés selon la présente invention sont les composés de formules (1) ou (2) présentant l'ensemble des caractéristiques suivantes :

- R est méthyle,
- B est méthyle,
- r = 0,
- s = 1,
- n = 0,
- m = 1.

Pour préparer les dérivés de formules (1) ou (2), on peut procéder classiquement (voie A) en mettant en oeuvre une réaction d'hydrosilylation à partir du dérivé siloxanique correspondant dans lequel, par exemple, tous les radicaux A sont des atomes d'hydrogène. Ce dérivé est dénommé par la suite dérivé à SiH.

Les groupes SiH peuvent être présents dans la chaîne et/ou aux extrémités de la chaîne. Ces dérivés à SiH sont des produits bien connus dans l'industrie des silicones et sont généralement disponibles dans le commerce. Ils sont par exemple décrits dans les brevets américains US-A-3 220 972, US-A-3 697 473 et US-A-4 340 709.

Les dérivés à SiH correspondant aux composés de formules (1) et (2) peuvent être donc représentés par les formules (4) et (5) suivantes :

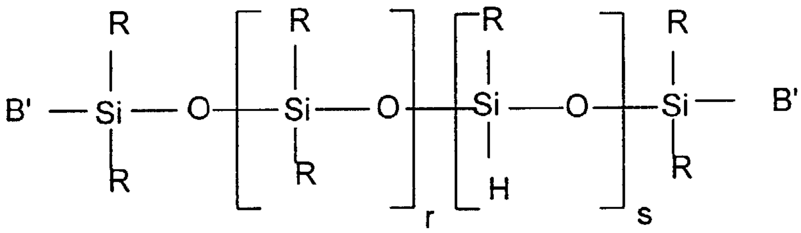 (4)

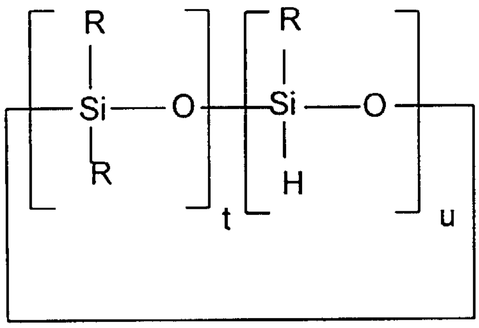 (5)

dans lesquelles :

- R, r, s, t et u ont la signification donnée ci-dessus pour les formules (1) et (2),
- B', identiques ou différents, sont choisis parmi les radicaux R et un atome d'hydrogène.

Sur ce dérivé à SiH de formules (4) ou (5), on effectue une réaction d'hydrosilylation en présence d'une quantité catalytiquement efficace d'un catalyseur au platine sur un dérivé organique choisi parmi ceux de formule (6) suivante :

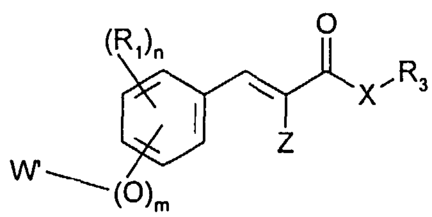

(6)

dans laquelle $R_1$, $R_3$, X, Z, m et n ont la même signification qu'à la formule (3) ci-dessus et dans laquelle W' a la même signification que W dans la formule (3) ci-dessus mais se termine soit par une double liaison, soit par une triple liaison.

La réaction d'hydrosilylation s'effectue donc selon l'une des deux réactions suivantes :

$$\equiv Si\text{-}H + CH_2{=}C\text{-} \text{ --------> } \equiv Si\text{-}CH_2\text{-}CH\text{-}$$

ou

$$\equiv Si\text{-}H + CH\equiv C\text{-} \text{ ---------> } \equiv Si\text{-}CH=CH\text{- et/ou } \equiv Si\text{-}C= CH_2$$

$$|$$

Les composés de formule (6) sont donc les composés suivants :

- 1°) les dérivés de benzalmalonates qui répondent aux formules suivantes :

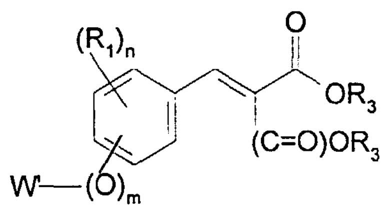

dans lesquelles $R_1$, R3, W', n et m ont les mêmes significations qu'à la formule (6) ci-dessus,

- 2°) les dérivés de benzalmalonamides qui répondent aux formules suivantes :

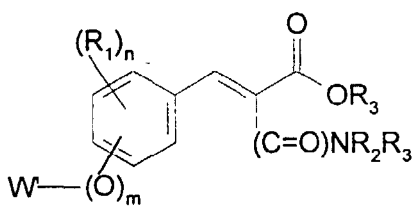

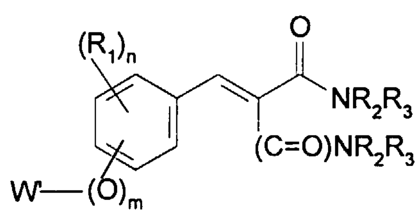

dans lesquelles $R_1$, $R_2$, $R_3$, n, m et W' ont les mêmes significations qu'à la formule (6) ci-dessus,

- 3°) les dérivés de cinnamamides qui répondent à la formule suivante :

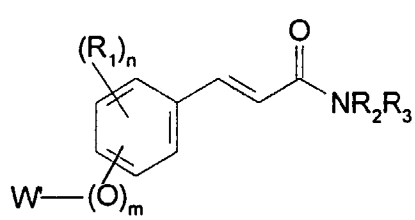

dans laquelle $R_1$, $R_2$, $R_3$, n, m et W' ont les mêmes significations qu'à la formule (3) ci-dessus.

Ainsi, comme dérivés de benzalmalonates convenant particulièrement bien à la préparation des composés selon l'invention, on peut citer le 4-méthallyloxy benzylidène malonate de diéthyle et le 4-allyloxy benzylidène malonate

de diéthyle qui sont décrits dans le brevet EP 392 882, le 4-(2-propynyloxy) benzylidène malonate de diéthyle décrit dans le brevet WO 92 20690.

Une autre voie de préparation (Voie B) des dérivés de formules (1) et (2) consiste, dans une première étape, à hydrosilyler un benzaldéhyde de formule (7) suivante :

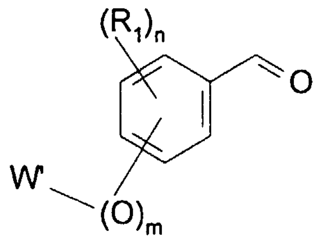

(7)

dans laquelle W', $R_1$, m et n ont la même signification qu'à la formule (6) ci-dessus,
par un composé de formule (4) ou (5) suivantes :

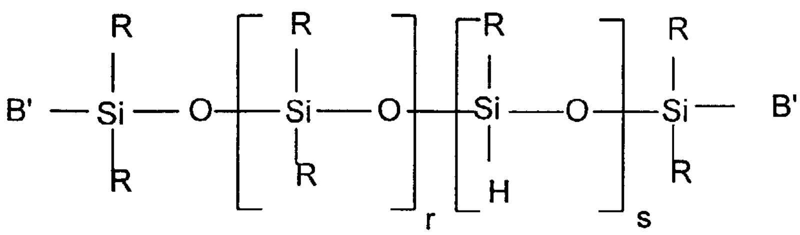

(4)

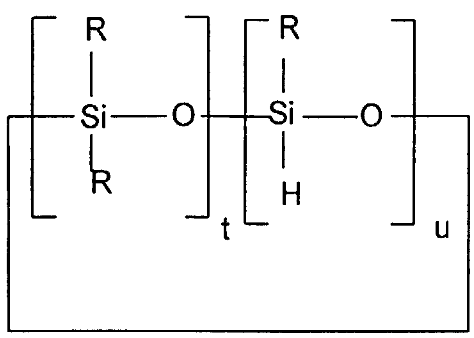

(5)

dans lesquelles :

- R, r, s, t et u ont la signification donnée ci-dessus pour les formules (1) et (2),
- B', identiques ou différents, sont choisis parmi les radicaux R et un atome d'hydrogène, pour obtenir le benzaldéhyde siliconé correspondant.

Dans une deuxième étape, sur ce benzaldéhyde siliconé correspondant, on fait réagir un dérivé organique choisi parmi ceux de formule (8) suivante :

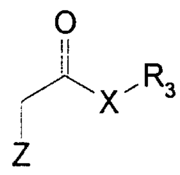

(8)

dans laquelle Z, X et $R_3$ ont la même signification qu'à la formule (6) ci-dessus.

Les composés de formule (8) dans le cas où X = O sont obtenus par alkylation de l'acide correspondant par un halogénure (Hal) d'alkyle en présence de base selon le schéma réactionnel suivant :

$$ZCH_2COOH + Hal\text{-}R_3 + NaOH \rightarrow (8) + Hal\text{-}Na$$

Les composés de formule (7) dans le cas ou X = $NR_3$ sont obtenus par réaction du chlorure d'acide correspondant par une amine selon le schéma réactionnel suivant :

$$ZCH_2COCl + H_2NR_2 \rightarrow (8) + HCl$$

Les composés de formule (7) dans le cas où m = 1 sont obtenus par alkylation du dérivé hydroxybenzaldéhyde correspondant par un halogénure de dérivé insaturé.

Les composés de formule (7) dans le cas où m = 0 sont connus en soi et ont été décrits dans la demande de brevet EP-A-350386 et préparés selon les synthèses définies dans cette référence. Ainsi, comme benzaldéhydes convenant particulièrement bien à la préparation des composés selon l'invention, on peut citer le 3-méthallyloxy benzaldéhyde, le 4-méthallyloxy benzaldéhyde qui sont décrits dans le brevet DE 2108932

La présente invention a également pour objet une composition comprenant un composé de formule (1) ou (2) selon l'invention dans un support approprié. Le support peut être par exemple une composition de matière plastique. Il peut également être approprié pour une application topique. Dans ce cas, la composition selon l'invention est une composition cosmétique qui comprend un support cosmétiquement acceptable.

De préférence, la composition selon l'invention est une composition destinée à protéger une matière sensible au rayonnement ultraviolet, en particulier au rayonnement solaire, comprenant une quantité efficace d'au moins un composé conforme à l'invention. Dans une forme préférée de réalisation de l'invention, cette composition est destinée à protéger la peau et/ou les cheveux.

Les composés de formule (1) ou (2) sont généralement présents dans la composition de l'invention dans des proportions comprises entre 0,1 % et 20 % en poids, de préférence entre 0,5 % et 10 % en poids, par rapport au poids total de la composition.

Les compositions selon l'invention peuvent bien entendu contenir un ou plusieurs filtres solaires complémentaires actifs dans l'UVA et/ou l'UVB (absorbeurs), autres que les composés conformes à la présente invention, hydrophiles ou lipophiles. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions selon l'invention peuvent encore contenir des pigments ou bien encore des nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Elle peut contenir les adjuvants cosmétiques habituellement utilisés dans le domaine cosmétique, tels que des corps gras, des solvants organiques, des silicones, des épaississants, des adoucissants, des filtres solaires complémentaires, des agents anti-mousses, des agents hydratants, des parfums, des conservateurs, des tensio-actifs,

des charges, des séquestrants, des polymères anioniques, cationiques, non ioniques ou amphotères ou leurs mélanges, des propulseurs, des agents alcalinisants ou acidifiants, des colorants, des pigments ou nanopigments en particulier ceux destinés à assurer un effet photoprotecteur complémentaire par blocage physique du rayonnement ultraviolet, ou tout autre ingrédient habituellement utilisé en cosmétique, en particulier pour la fabrication des compositions antisolaires.

Parmi les solvants organiques, on peut citer les alcools et polyols inférieurs tels que l'éthanol, l'isopropanol, le propylèneglycol, la glycérine et le sorbitol.

Les corps gras peuvent être constitués par une huile ou par une cire ou leurs mélanges, des acides gras, des esters d'acides gras, des alcools gras, la vaseline, la paraffine, la lanoline, la lanoline hydrogénée, la lanoline acétylée. Les huiles peuvent être choisies parmi les huiles animales, végétales, minérales ou de synthèse, et notamment l'huile de palme hydrogénée, l'huile de ricin hydrogénée, l'huile de vaseline, l'huile de paraffine, l'huile de Purcellin, les huiles de silicones, volatiles ou non, et les isoparaffines.

Bien entendu, l'homme de l'art veillera à choisir le ou les éventuels composés complémentaires cités ci-dessus et/ou leurs quantités de manière telle que les propriétés avantageuses attachées intrinsèquement au composé conforme à l'invention ne soient pas, ou substantiellement pas, altérées par la ou les adjonctions envisagées.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de crème, de lait, de poudre, de bâtonnet solide et eventuellement être conditionnée en aérosol et se présenter sous forme de mousse ou de spray.

Lorsque la composition cosmétique selon l'invention est plus particulièrement destinée à la protection de l'épiderme humain contre les rayons UV ou comme composition antisolaire, elle peut se présenter sous forme de suspension ou de dispersion dans des solvants ou des corps gras, ou encore sous forme d'émulsion (notamment de type H/E ou E/H, mais de préférence H/E) telle qu'une crème ou un lait, de dispersion vésiculaire, sous forme de pommade, de gel, de bâtonnet solide ou de mousse aérosol. Les émulsions peuvent contenir en outre des agents tensio-actifs anioniques, non ioniques, cationiques ou amphotères.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux.

Lorsque la composition cosmétique selon l'invention est utilisée comme produit de maquillage des cils, des sourcils, de la peau ou des cheveux, tels que crème de traitement de l'épiderme, fond de teint, bâton de rouge à lèvres, fards à paupière, fards à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a encore pour objet l'utilisation d'un composé conforme à l'invention dans des, ou pour la fabrication de, compositions destinées à protéger des matières sensibles au rayonnement ultraviolet, en particulier au rayonnement solaire.

L'invention a encore pour objet l'utilisation d'un composé de formule (1) ou (2) conforme à l'invention pour la préparation d'un médicament destiné à prévenir les effets néfastes des rayonnements UV.

L'invention a encore pour objet l'utilisation d'un composé de formule (1) ou (2), conforme à l'invention à titre d'agent filtrant les rayonnements UV, en particulier pour contrôler la couleur de la peau.

L'invention a enfin pour objet un procédé non thérapeutique de protection de la peau et/ou des cheveux contre le rayonnement ultraviolet, en particulier le rayonnement solaire, qui consiste à appliquer sur la peau ou les cheveux une quantité efficace de la composition cosmétique définie ci-dessus, ou d'un composé de formule (1) ou (2), tel que défini ci-avant.

L'invention a finalement pour objet un procédé non thérapeutique de contrôle de la variation de couleur de la peau due aux rayonnements UV, qui consiste à appliquer sur la peau une quantité efficace de la composition cosmétique définie ci-dessus, ou d'un composé de formule (1) ou (2), tel que défini ci-avant.

Les exemples qui suivent illustrent l'invention sans toutefois en limiter la portée.

**EXEMPLE 1 :**

Préparation du 4-[2-méthyl-3-[1,3,3,3-tetraméthyl-1-[(triméthylsilyl)oxy]-disiloxanyl]propyloxy]-benzylidène malonate de diéthyle selon la voie A :

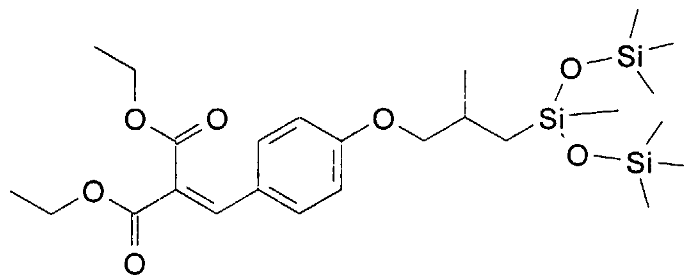

A une solution de 4-méthallyloxy benzylidène malonate de diéthyle (15,9 g, 0,05 mole) et de catalyseur (complexe à 3-3.5% poids de Pt dans du cyclovinylméthylsiloxane de Hüls Petrarch PC085 : 100 µl) dans 30 ml de toluène sec porté à 70°C, on ajoute goutte à goutte en 30 minutes 12,23 g (0,055 mole) d'heptaméthyltrisiloxane. On laisse à cette température pendant 6 heures. On concentre le mélange réactionnel et on obtient après chromatographie sur silice (éluant : heptane/acétate d'éthyle 95:5) 14,8 g (Rendement : 55 %) d'une huile incolore ayant les caractéristiques suivantes :

-UV (Ethanol) $\lambda_{max}$ = 314 nm, $\varepsilon_{max}$ = 25 000

| Analyse élémentaire pour $C_{25}H_{44}O_7Si_3$ | | | |
|---|---|---|---|
| théorie | C : 55.52 | H : 8.20 | Si : 15.58 |
| trouvé | C : 55.31 | H : 8.16 | Si : 15.29 |

**EXEMPLE 2 :**

Préparation du 3-[2-méthyl-3-[1,3,3,3-tetraméthyl-1-[(triméthylsilyl)oxy]-disiloxanyl] propyloxy]-benzylidène malonate de diéthyle selon la voie B :

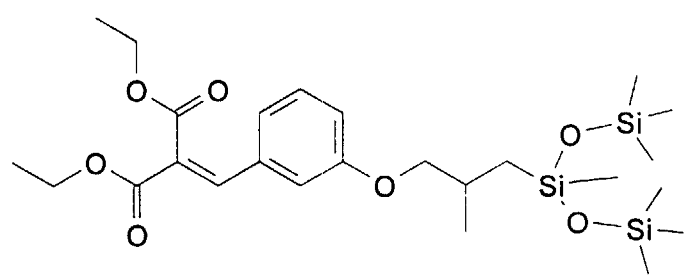

a) Première étape : Préparation du 3-[2-méthyl-3-[1,3,3,3-tetraméthyl-1-[(triméthylsilyl) oxy]disiloxanyl]propyloxy]-benzaldéhyde :

A une solution de 3-méthallyloxy benzaldéhyde (20 g, 0,113 mole) et de catalyseur (complexe à 3-3.5% poids de Pt dans du cyclovinylméthylsiloxane de Hüls Petrarch PC085 : 200 µl) dans 40 ml de toluène sec porté à 80°C, on ajoute goutte à goutte en 30 minutes 12,23 g (0,055 mole) d'heptaméthyltrisiloxane. On laisse à cette température pendant 16 heures. On concentre le mélange réactionnel et on obtient après chromatographie sur silice (éluant : heptane/acétate d'éthyle 90:10) 26,4 g (Rendement : 59 %) d'une huile incolore de 3-[2-méthyl-3-[1,3,3,3-tetraméthyl-1-[(triméthylsilyl)oxy] disiloxanyl]propyloxy]-benzaldéhyde.

b) Deuxième étape : Préparation du 3-[2-méthyl-3-[1,3,3,3-tetraméthyl-1-[(triméthylsilyl)oxy]disiloxanyl]propyloxy]-benzylidène malonate de diéthyle :

On porte au reflux pendant 3 heures un mélange du dérivé précédent (4 g, 0,0085 mole) et de malonate de diéthyle (1,6 g, 0,01 mole) dans 15 ml de toluène en présence de 0,1 ml de pipéridine et de 0,06 ml d'acide acétique. On concentre le mélange réactionnel et on obtient après chromatographie sur silice (éluant : heptane/dichlorométhane 50:50) 3,5 g (Rendement: 76 %) d'une huile incolore ayant les caractéristiques suivantes :

-UV (Ethanol) $\lambda_{max}$ = 280 nm, $\varepsilon_{max}$ = 16 700

$\lambda_{max}$ = 320 nm, $\varepsilon_{max}$ = 3 400

| Analyse élémentaire pour $C_{25}H_{44}O_7Si_3$ | | |
|---|---|---|
| théorie | C : 55.52 | H : 8.20 |
| trouvé | C : 55.77 | H : 8.01 |

**EXEMPLE 3 :**

Préparation du 3-[4-[2-méthyl-3-[1,3,3,3-tetraméthyl-1-[(triméthylsilyl)oxy]-disiloxanyl] propyloxy]-phenyl]-2-(piperidine-1-carbonyl)-acrylic acid ethyl ester selon la voie B :

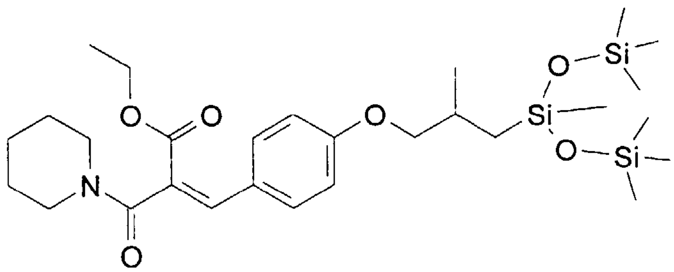

Première étape : Préparation du 4-[2-méthyl-3-[1,3,3,3-tetraméthyl-1-[(triméthylsilyl)oxy]disiloxanyl]propyloxy]-benzaldéhyde :

A une solution de 4-méthallyloxy benzaldéhyde (17,62 g, 0,1 mole) et de catalyseur (complexe à 3-3.5% poids de Pt dans du cyclovinylméthylsiloxane de Hüls Petrarch PC085 : 300 µl) dans 50 ml de toluène sec porté à 80°C, on ajoute goutte à goutte en 30 minutes 24,47 g (0,11 mole) d'heptaméthyltrisiloxane. On laisse à cette température pendant 26 heures. On concentre le mélange réactionnel et on obtient après distillation sous pression de 0,1 mmHg (fractions distillant à 135-141 °C), 34,1 g de 4-[2-méthyl-3-[1,3,3,3-tetraméthyl-1-[(triméthylsilyl)oxy]disiloxanyl] propyloxy]-benzaldéhyde (Rendement : 85 %) sous forme d'une huile incolore.

b) Deuxième étape : Préparation du 3-[4-[2-méthyl-3-[1,3,3,3-tetraméthyl-1-[(triméthylsilyl)oxy]-disiloxanyl]propyloxy]-phenyl]-2-(piperidine-1-carbonyl)-acrylic acid ethyl ester :

On porte au reflux pendant 7 heures un mélange du dérivé précédent (18,18 g, 0,046 mole) et de malonate de diéthyle (9,61 g, 0,06 mole) dans 20 ml de toluène en présence de 4,5 ml de pipéridine et de 1,5 ml d'acide acétique. On concentre le mélange réactionnel et on obtient après chromatographie sur silice (éluant : heptane/dichlorométhane 95:5) des dernières fractions 8 g (Rendement : 30 %) d'une huile incolore visqueuse ayant les caractéristiques suivantes :

-UV (Ethanol) $\lambda_{max}$ = 315 nm, $\varepsilon_{max}$ = 19 860

**EXEMPLE 4 :**

Préparation du 4-[3-[1,3,3,3-tetraméthyl-1-[(triméthylsilyl)oxy]disiloxanyl]-propyloxy]-benzylidène malonate de diéthyle selon la voie A :

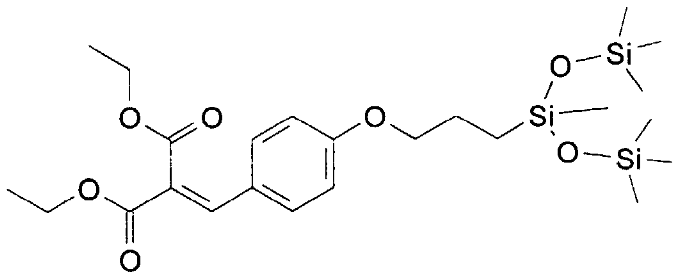

A un mélange de 4-allyloxy benzylidène malonate de diéthyle (17,7 g, 0,058 mole) et de catalyseur (complexe à 3-3.5% poids de Pt dans du cyclovinylméthylsiloxane de Hüls Petrarch PC085 : 400 μl) porté à 60°C, on ajoute goutte à goutte en 30 minutes 14,23 g (0,064 mole) d'heptaméthyltrisiloxane. On laisse à cette température pendant 1 heure. On élimine sous vide l'excès d'heptaméthyltrisiloxane. On purifie par une chromatographie sur silice (éluant : heptane/acétate d'éthyle 96:4) pour obtenir 11,4 g (Rendement : 37 %) de fractions propres d'une huile incolore ayant les caractéristiques suivantes :

-UV (Ethanol) $\lambda_{max}$ = 314 nm, $\varepsilon_{max}$ = 25 420

| Analyse élémentaire pour $C_{24} H_{42} O_7 Si_3$ | | |
|---|---|---|
| théorie | C : 54.71 | H : 8.04 |
| trouvé | C . 54.84 | H : 8.11 |

**EXEMPLE 5 :**

Préparation du 4-[3-[1,3,3,3-tetraméthyl-1-[(triméthylsilyl)oxy]disiloxanyl]-propyloxy]-benzylidène malonate de diméthyle selon la voie A :

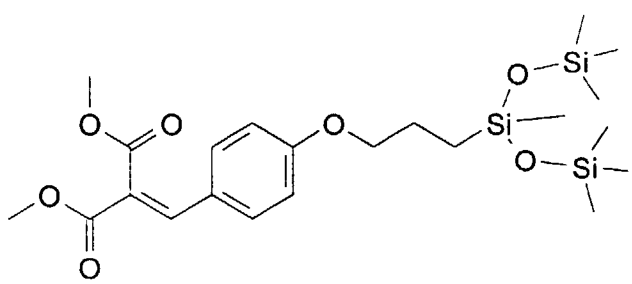

A un mélange de 4-allyloxy benzylidène malonate de diméthyle (8,55 g, 0,031 mole) et de catalyseur (complexe à 3-3.5% poids de Pt dans du cyclovinylméthylsiloxane de Hüls Petrarch PC085 : 100 μl) dans 10 ml de toluène porté à 60°C, on ajoute goutte à goutte en 30 minutes 7,97 g (0,034 mole) d'heptaméthyltrisiloxane. On laisse à cette température pendant 6 heures. On élimine sous vide l'excès d'heptaméthyltrisiloxane. On purifie par une chromatographie sur silice (éluant : heptane/acétate d'éthyle 96:4) pour obtenir 1,5 g (Rendement : 11 %) de fractions propres d'une huile incolore ayant les caractéristiques suivantes :

-UV (Ethanol) $\lambda_{max}$ = 315 nm, $\varepsilon_{max}$ = 25 640

| Analyse élémentaire pour $C_{22}$ $H_{38}$ $O_7$ $Si_3$ | | |
|---|---|---|
| théorie | C : 52.98 | H : 7.68 |
| trouvé | C : 52.64 | H : 7.59 |

**EXEMPLE 6 :**

Préparation du 4-[3-[1,3,3,3-tetraméthyl-1-[(triméthylsilyl)oxy]disiloxanyl]-2-propenyloxy]-benzylidène malonate de diéthyle et du 4-[3-[1,3,3,3-tetraméthyl-1-[(triméthylsilyl)oxy]disiloxanyl]-2-vinylidène-ethanoxy]-benzylidène malonate de diéthyle :

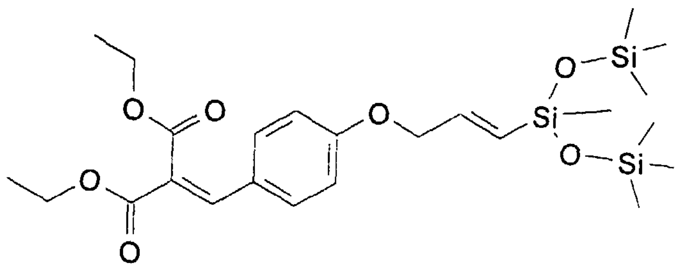

90 %

+

/

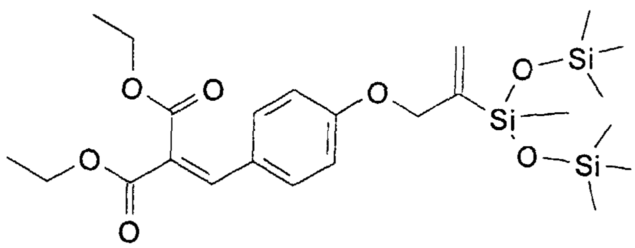

10 %

A un mélange de 4-(2-propynyloxy) benzylidène malonate de diéthyle (15,1 g, 0,05 mole) et de catalyseur (complexe à 3-3.5% poids de Pt dans du cyclovinylméthylsiloxane de Hüls Petrarch PC085 : 100 µl) dans 30 ml de toluène porté à 90°C, on ajoute goutte à goutte en 30 minutes 12,2 g (0,055 mole) d'heptaméthyltrisiloxane. On laisse à cette température pendant 1 heure 30 minutes. On élimine sous vide l'excès d'heptaméthyltrisiloxane. On purifie par une chromatographie sur silice (éluant : $CH_2Cl_2$) pour obtenir 12,1 g (Rendement : 46 %) de fractions propres d'une huile incolore. La RMN du proton montre que l'on obtient un mélange de 2 produits dans le rapport 9:1 :

-UV (Ethanol) $\lambda_{max}$ = 313 nm, $\varepsilon_{max}$ = 26 530

| Analyse élémentaire pour $C_{22}$ $H_{38}$ $O_7$ $Si_3$ | | | |
|---|---|---|---|
| théorie | C : 52.98 | H : 7.68 | Si : 16.89 |
| trouvé | C : 52.62 | H : 7.72 | Si : 16.80 |

**EXEMPLE 7 :**

On donne ci-après un exemple concret d'une émulsion solaire de composition suivante (les quantités sont exprimées en % de poids par rapport au poids total de la composition) :

- mélange d'alcool cétylstéarylique et d'alcool cétylstéarylique oxyéthyléné (33 OE) 80/20 vendu sous la dénomination commerciale commerciale « DEHSCONET 390 » par TENSIA 7 %

- mélange de mono et distéarate de glycérol vendu sous la dénomination commerciale « CERASYNTH SD » par la société ISP 2 %
- alcool cétylique 1,5 %
- polydiméthylsiloxane vendu sous la dénomination commerciale « DC200 Fluid » par la société DOW CORNING 1,5 %
- benzoate d'alcools en $C_{12}$-$C_{15}$ vendu sous la dénomination commerciale « FINSOLV TN » par la société FINETEX 15 %
- composé de l'exemple 1 5 %
- glycérine 20 %
- conservateurs qs
- eau déminéralisée qsp 100 %

Cette émulsion H/E solaire est préparée selon les techniques classiques de préparation d'émulsions en dissolvant le filtre dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40°C on ajoute enfin les conservateurs.
On obtient ainsi une crème antisolaire particulièrement efficace contre les rayonnements UV.

## Revendications

1. Composé répondant à l'une des deux formules (1) ou (2) suivantes :

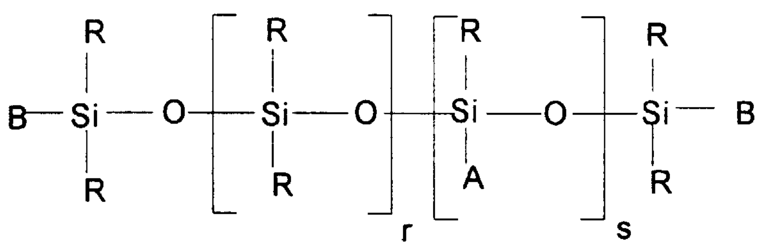

(1)

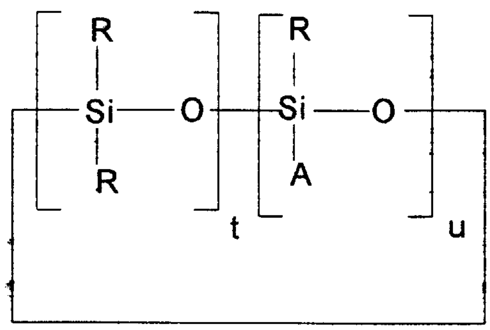

(2)

dans lesquelles :

- R, identiques ou différents sont choisis parmi les radicaux alkyles en $C_1$-$C_{10}$, linéaires ou ramifiés, saturés ou insaturés, le radical phényle et le radical trifluoro-3,3,3 propyle, au moins 80% en nombre des radicaux R étant le radical méthyle,
- B, identiques ou différents, sont choisis parmi les radicaux R et le radical A,
- r est un nombre entier choisi entre 0 et 3 inclusivement,

- s est 0 ou 1 et si s est 0, au moins un des deux symboles B est A,
- u est égal à 1 ou 2,
- t est un nombre entier entre 0 et 5 inclus,
- t + u est supérieur ou égal à 3,

et A est un radical de formule (3) suivante:

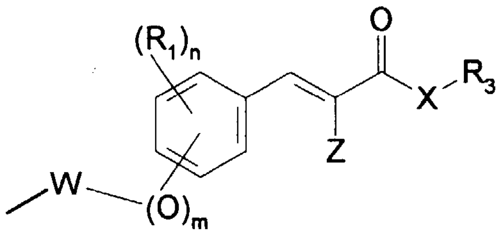

(3)

dans laquelle :

- $R_1$ représente un radical alcoxy en $C_1$-$C_{10}$ linéaire ou ramifié, un radical alkyle en $C_1$-$C_{10}$ linéaire ou ramifié, un radical alcènyle en $C_2$-$C_8$ linéaire ou ramifié ou -$OSi(CH_3)_3$, deux $R_1$ adjacents pouvant former ensemble un groupement alkylidène dioxy dans lequel le groupe alkylidène contient de 1 à 2 atomes de carbone,
- n est 0, 1 ou 2,
- m est 0 ou 1,
- Z est l'hydrogène ou un radical -(C=O)$YR_3$,
- X et Y, identiques ou différents, représentent indépendamment l'oxygène ou un radical -$NR_2$-
- W est un radical alcane di-yle en $C_1$-$C_6$ linéaire ou ramifié, saturé ou insaturé, éventuellement substitué par un radical hydroxyle ou alcoyle en $C_2$-$C_8$, linéaire ou ramifié, saturé ou insaturé,
- $R_2$ est indépendamment l' hydrogène ou un radical alkyle en $C_1$-$C_8$,
- $R_3$ est indépendamment un radical alkyle en $C_1$-$C_{12}$ linéaire ou ramifié,
- $R_2$ et $R_3$, pris ensemble, pouvant former un hétérocycle,

avec la condition que lorsque Z = H, alors X est le radical -$NR_2$-.

**2.** Composé selon la revendication 1, caractérisé par le fait qu'il présente au moins l'une des caractéristiques suivantes :

- R est méthyle,
- B est méthyle,
- r est 0 ou 1,
- s est 1,

- t + u est compris entre 3 et 5,
- n est 0 ou 1,
- $R_1$ est méthoxy,
- $R_3$ est méthyle ou éthyle,
- m est 1.

**3.** Composé selon la revendication 1 ou 2, caractérisé par le fait qu'il présente l'ensemble des caractéristiques suivantes :

- R est méthyle,
- B est méthyle,
- r = 0,
- s = 1,
- n = 0,
- m = 1.

**4.** Composé selon l'une quelconque des revendications 1 à 3, caractérisé par le fait qu'il est choisi parmi :

- le 4-[2-méthyl-3-[1,3,3,3-tetraméthyl-1-[(triméthylsilyl)oxy]-disiloxanyl]propyloxy]-benzylidène malonate de diéthyle,
- le 3-[2-méthyl-3-[1,3,3,3-tetraméthyl-1-[(triméthylsilyl)oxy]-disiloxanyl]propyloxy]-benzylidène malonate de diéthyle,
- le 3-[4-[2-méthyl-3-[1,3,3,3-tetraméthyl-1-[(triméthylsilyl)oxy]-disiloxanyl]-propyloxy]phenyl]-2-(piperidine-1-carbonyl)-acrylic acid ethyl ester,
- le 4-[3-[1,3,3,3-tetraméthyl-1-[(triméthylsilyl)oxy]disiloxanyl]-propyloxy]-benzylidène malonate de diéthyle,
- le 4-[3-[1,3,3,3-tetraméthyl-1-[(triméthylsilyl)oxy]disiloxanyl]-propyloxy]-benzylidène malonate de diméthyle,
- le 4-[3-[1,3,3,3-tetraméthyl-1-[(triméthylsilyl)oxy]disiloxanyl]-2-propenyloxy]-benzylidène malonate de diéthyle,
- le 4-[3-[1,3,3,3-tetraméthyl-1-[(triméthylsilyl)oxy]disiloxanyl]-2-vinylidène-ethanoxy]-benzylidène malonate de diéthyle.

**5.** Procédé de préparation d'un composé tel que défini à l'une quelconque des revendications 1 à 4, caractérisé par le fait qu'il comprend l'étape suivante :

- on effectue, en présence d'une quantité catalytiquement efficace d'un catalyseur au platine, l'hydrosilylation d'un composé de formule (6) suivante :

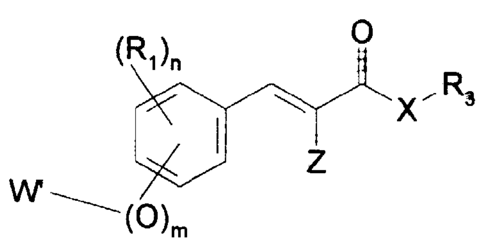

(6)

dans laquelle $R_1$, $R_3$, X, Z et n ont la même signification qu'à la formule (3) de la revendication 1 et dans laquelle W' a la même signification que W dans la formule (3) de la revendication 1 mais se termine soit par une double liaison, soit par une triple liaison,

par un dérivé de formule (4) ou (5) suivantes :

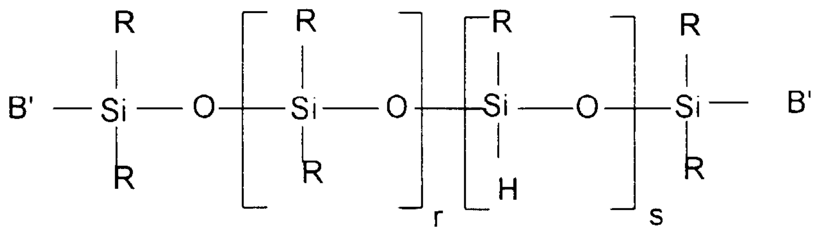

(4)

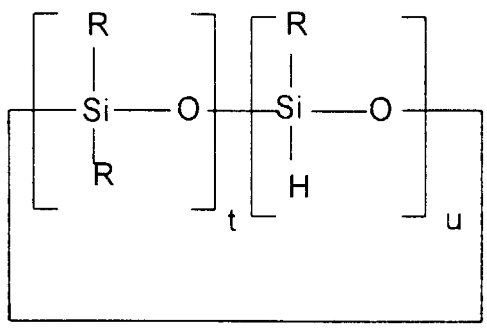

(5)

dans lesquelles :

- R, r, s, t et u ont la signification donnée à la revendication 1 pour les formules (1) et (2),
- B', identiques ou différents, sont choisis parmi les radicaux R et un atome d'hydrogène.

**6.** Procédé de préparation d'un composé de formule (1) ou (2), caractérisé par le fait qu'il comprend les étapes suivantes :

i) on fait réagir un benzaldéhyde de formule (7) suivante :

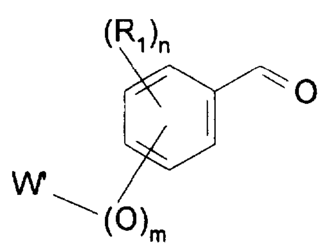

(7)

dans laquelle W', $R_1$, m et n ont la même signification qu'à la formule (6) de la revendication 5, et un composé de formule (4) ou (5) suivantes :

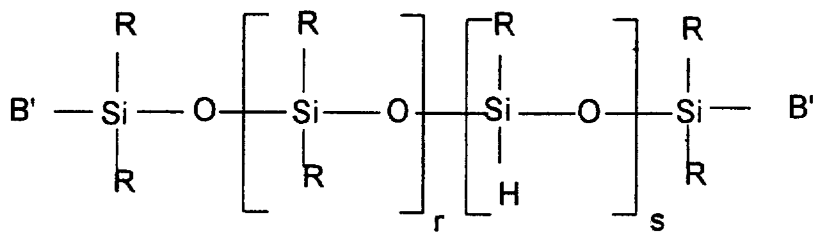

(4)

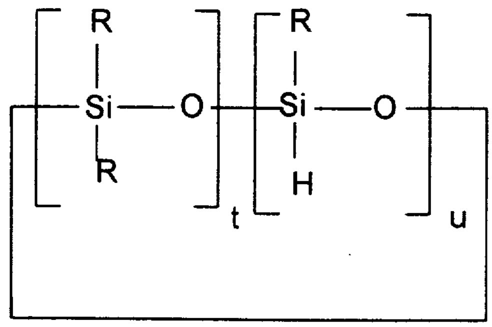

(5)

dans lesquelles :

- R, r, s, t et u ont la signification donnée ci-dessus pour les formules (1) et (2),
- B', identiques ou différents, sont choisis parmi les radicaux R et un atome d'hydrogène,

pour obtenir le benzaldéhyde siliconé correspondant,

ii) sur le benzaldéhyde siliconé obtenu à l'étape i), on fait réagir un dérivé organique choisi parmi ceux de formule (8) suivante :

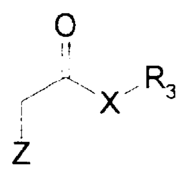

(8)

dans laquelle Z, X et $R_3$ ont la même signification qu'à la formule (6) de la revendication 5.

**7.** Composition de matière plastique, caractérisée par le fait qu'elle comprend au moins un composé tel que défini à l'une quelconque des revendications 1 à 4.

**8.** Composition cosmétique destinée à protéger la peau et/ou les cheveux du rayonnement UV comprenant dans un support cosmétiquement acceptable au moins un composé tel que défini à l'une quelconque des revendications 1 à 4.

**9.** Composition selon la revendication 8, caractérisée par le fait que le composé tel que défini à l'une quelconque des revendications 1 à 4 est présent dans la composition à une teneur allant de 0,1 à 20 % en poids, par rapport au poids total de la composition, de préférence allant de 0,5 % à 10 % en poids, par rapport au poids total de la composition.

**10.** Utilisation d'au moins un composé tel que défini à l'une quelconque des revendications 1 à 4 pour la fabrication de compositions destinées à protéger des matières sensibles au rayonnement ultraviolet, en particulier au rayonnement solaire.

**11.** Utilisation d'au moins un composé tel que défini à l'une quelconque des revendications 1 à 4 à titre d'agent filtrant les rayonnements UV pour la fabrication de compositions cosmétiques destinées à protéger la peau et/ou les cheveux du rayonnement UV

**12.** Utilisation d'au moins un composé tel que défini à l'une quelconque des revendications 1 à 4 à titre d'agent filtrant les rayonnements UV pour la préparation d'un médicament destiné à prévenir les effets néfastes des rayonnements UV.

**13.** Utilisation d'un composé tel que défini à l'une quelconque des revendications 1 à 4 à titre d'agent pour contrôler la couleur de la peau pour la fabrication de compositions cosmétiques.

## Patentansprüche

**1.** Verbindung, die einer der beiden folgenden Formeln (1) oder (2) entspricht:

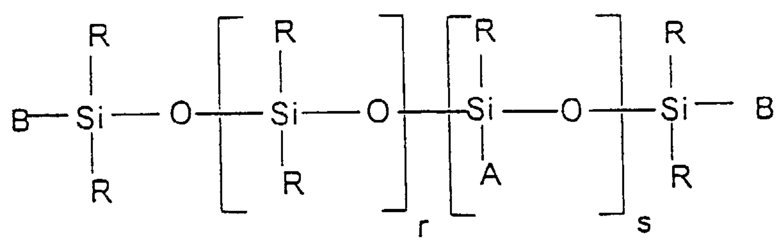

(1)

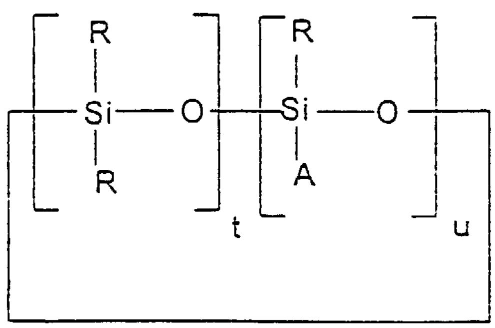

(2)

worin:

- die Gruppen R, die identisch oder voneinander verschieden sind, unter den geradkettigen oder verzweigten, gesättigten oder ungesättigten $C_{1-10}$-Alkylgruppen, Phenyl oder 3,3,3-Trifluorpropyl ausgewählt sind, wobei mindestens 80 % der Gruppen R Methyl bedeuten,
- die Gruppen B, die identisch oder voneinander verschieden sind, unter den Gruppen R und der Gruppe A ausgewählt sind,
- r 0 oder eine ganze Zahl im Bereich von 1 bis 3 bedeutet,
- s 0 oder 1 bedeutet, wobei mindestens eine der beiden Gruppen B A bedeutet, wenn s 0 ist,
- u 1 oder 2 bedeutet,
- t 0 oder eine ganze Zahl im Bereich von 1 bis 5 ist,
- $t + u \geq 3$ ist, und
- A eine Gruppe der folgenden Formel (3) ist

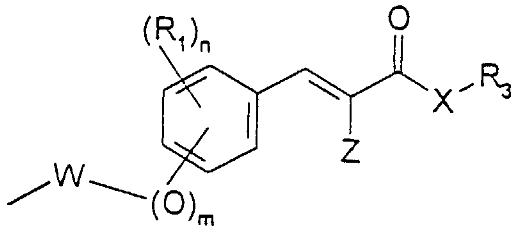

(3),

worin bedeuten:

- $R_1$ eine geradkettige oder verzweigte $C_{1-10}$-Alkoxygruppe, eine geradkettige oder verzweigte $C_{1-10}$-Alkylgruppe, eine geradkettige oder verzweigte $C_{2-8}$-Alkenylgruppe oder $-OSi(CH_3)_3$, wobei zwei angrenzende Gruppen $R_1$ gemeinsam eine Alkylidendioxygruppe bilden können, worin die Alkylidengruppe 1 bis 2 Kohlenstoffatome aufweist,
- n 0, 1 oder 2,
- m 0 oder 1,
- Z Wasserstoff oder eine Gruppe $-(C{=}O)YR_3$,
- die Gruppen X und Y, die identisch oder voneinander verschieden sind, unabhängig voneinander Sauerstoff oder eine Gruppe $-NR_2-$,
- W eine geradkettige oder verzweigte, gesättigte oder ungesättigte $C_{1-6}$-Alkandiylgruppe, die gegebenenfalls mit einer Hydroxygruppe oder einer geradkettigen oder verzweigten, gesättigten oder ungesättigten $C_{2-8}$-Alkylgruppe substituiert ist,
- $R_2$ Wasserstoff oder eine $C_{1-8}$-Alkylgruppe,
- $R_3$ eine geradkettige oder verzweigte $C_{1-12}$-Alkylgruppe,
- wobei $R_2$ und $R_3$ gemeinsam einen Heterocyclus bilden können,

mit der Maßgabe, daß X die Gruppe $-NR_2-$ bedeutet, wenn Z = H ist.

**2.** Verbindung nach Anspruch 1, dadurch gekennzeichnet, daß sie mindestens eine der folgenden Eigenschaften aufweist:

- R ist Methyl,
- B ist Methyl,
- r ist 0 oder 1,
- s ist 1,
- t + u liegt im Bereich von 3 bis 5,
- n ist 0 oder 1,
- $R_1$ ist Methoxy,
- $R_3$ ist Methyl oder Ethyl,
- m ist 1.

**3.** Verbindung nach Anspruch 1 oder 2, dadurch gekennzeichnet, daß sie die gesamten folgenden Eigenschaften aufweist:

- R ist Methyl,
- B ist Methyl,
- r = 0,
- s = 1.
- n = 0,
- m = 1.

**4.** Verbindung nach einem der Ansprüche 1 bis 3, dadurch gekennzeichnet, daß sie ausgewählt ist unter:

Diethyl-4-[2-methyl-3-[1,3,3,3-tetramethyl-1-[(trimethylsilyl)oxy]disiloxanyl]-propyloxy]-benzylidenmalonat,
Diethyl-3-[2-methyl-3-[1,3,3,3-tetramethyl-1-[(trimethylsilyl)oxy]disiloxanyl]-propyloxy]-benzylidenmalonat,
3-[4-[2-Methyl-3-[1,3,3,3-tetramethyl-1-[(trimethylsilyl)oxy]disiloxanyl]-propyloxy]-phenyl]-2-(piperidin-1-carbonyl)-acrylsäureethylester,
Diethyl-4-[3-[1,3,3,3-tetramethyl-1-[(trimethylsilyl)-oxy]disiloxanyl]-propyloxy]-benzylidenmalonat,
Dimethyl-4-[3-[1,3,3,3-tetramethyl-1-[(trimethylsilyl)-oxy]disiloxanyl]-propyloxy]-benzylidenmalonat,
Diethyl-4-[3-[1,3,3,3-tetramethyl-1-[(trimethylsilyl)-oxy]disiloxanyl]-2-propenyloxy]-benzylidenmalonat,
Diethyl-4-[3-[1,3,3,3-tetramethyl-1-[(trimethylsilyl)-oxy]disiloxanyl]-2-vinylidenethanoxy]-benzylidenmalonat.

**5.** Verfahren zur Herstellung einer Verbindung nach einem der Ansprüche 1 bis 4, dadurch gekennzeichnet, daß es den folgenden Schritt umfaßt:

- Durchführung einer Hydrosilylierung mit einer Verbindung der folgenden Formel (6) :

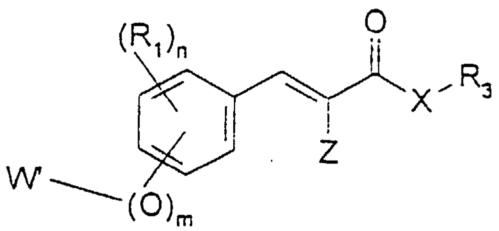

(6)

worin $R_1$, $R_3$, X, Z und n die für Formel (3) in Anspruch 1 angegebenen Bedeutungen aufweisen und worin W' die für W in Formel (3) gemäß Anspruch 1 angegebene Bedeutung aufweist, jedoch entweder mit einer Doppelbindung oder mit einer Dreifachbindung abschließt,

und einem Derivat der folgenden Formel (4) oder (5) :

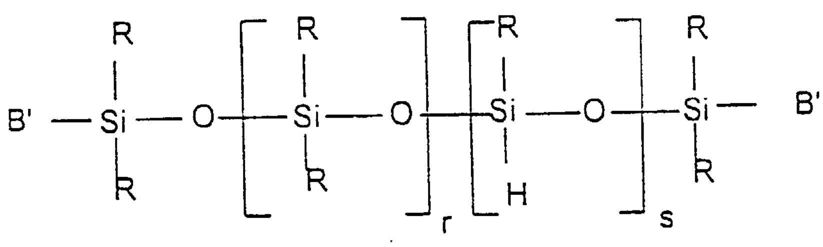

(4)

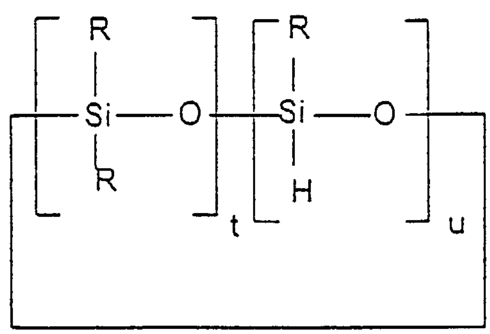

(5)

worin bedeuten:

- R, r, s, t und u die in Anspruch 1 für die Formel (1) und (2) angegebenen Bedeutungen und
- die Gruppen B', die identisch oder voneinander verschieden sind, die Gruppen R oder Wasserstoff, in Gegenwart einer katalytisch wirksamen Menge eines Platinkatalysators.

**6.** Verfahren zur Herstellung einer Verbindung der Formel (1) oder (2), dadurch gekennzeichnet, daß sie die folgenden Schritte umfaßt:

i) Umsetzung eines Benzaldehyds der folgenden Formel (7) :

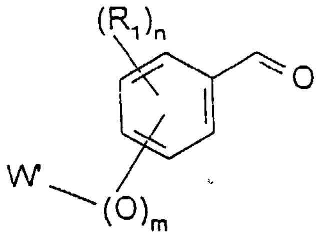

(7)

worin W', $R_1$, m und n die für Formel (6) in Anspruch 5 angegebenen Bedeutungen aufweisen, mit einer Verbindung der folgenden Formel (4) oder (5):

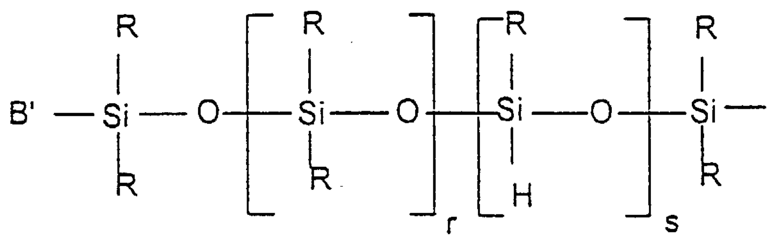

(4)

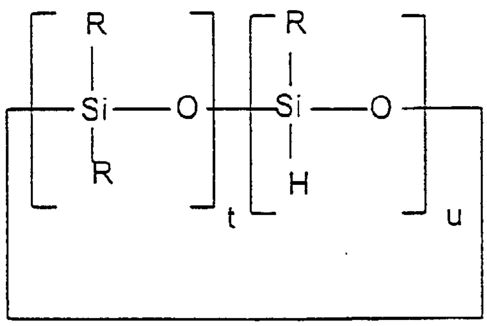

(5) ,

worin bedeuten:

- R, r, s, t und u die oben für die Formel (1) und (2) angegebenen Bedeutungen und
- die Gruppen B', die identisch oder voneinander verschieden sind, die Gruppen R oder Wasserstoff, zur Herstellung des entsprechenden siliconhaltigen Benzaldehyds, und

ii) Umsetzung eines organischen Derivats mit dem in Schritt i) hergestellten siliconhaltigen Benzaldehyd, wobei das organische Derivat unter den Derivaten der folgenden Formel (8) ausgewählt ist:

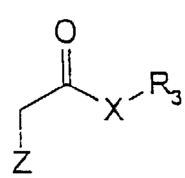

(8)

worin Z, X und $R_3$ die für Formel (6) in Anspruch 5 angegebenen Bedeutungen aufweisen.

**7.** Zusammensetzung aus Kunststoffmaterial, dadurch gekennzeichnet, daß sie mindestens eine Verbindung nach einem der Ansprüche 1 bis 4 enthält.

**8.** Kosmetische Zusammensetzung zum Schutz der Haut und/oder der Haare gegen UV-Strahlung, die in einem kosmetisch akzeptablen Träger mindestens eine Verbindung nach einem der Ansprüche 1 bis 4 enthält.

**9.** Zusammensetzung nach Anspruch 8, dadurch gekennzeichnet, daß die Verbindung nach einem der Ansprüche 1 bis 4 in der Zusammensetzung in einem Mengenanteil von 0,1 bis 20 Gew.-%, bezogen auf das Gesamtgewicht der Zusammensetzung, und vorzugsweise im Bereich von 0,5 bis 10 Gew.-%, bezogen auf das Gesamtgewicht der Zusammensetzung, vorliegt.

**10.** Verwendung mindestens einer Verbindung nach einem der Ansprüche 1 bis 4 zur Herstellung von Zusammensetzungen, die dazu dienen sollen, gegenüber UV-Strahlung und insbesondere gegenüber Sonnenlicht empfindliche Materialien zu schützen.

**11.** Verwendung mindestens einer Verbindung nach einem der Ansprüche 1 bis 4 als UV-Filter zur Herstellung von kosmetischen Zusammensetzungen, die zum Schutz der Haut und/oder der Haare gegen UV-Strahlung dienen sollen.

**12.** Verwendung mindestens einer Verbindung nach einem der Ansprüche 1 bis 4 als UV-Filter zur Herstellung eines Arzneimittels, das den schädlichen Wirkungen der UV-Strahlung vorbeugen soll.

**13.** Verwendung einer Verbindung nach einem der Ansprüche 1 bis 4 als Mittel zur Regulierung der Hautfarbe zur Herstellung von kosmetischen Zusammensetzungen.

## Claims

**1.** Compound corresponding to one of the two formulae (1) or (2) below:

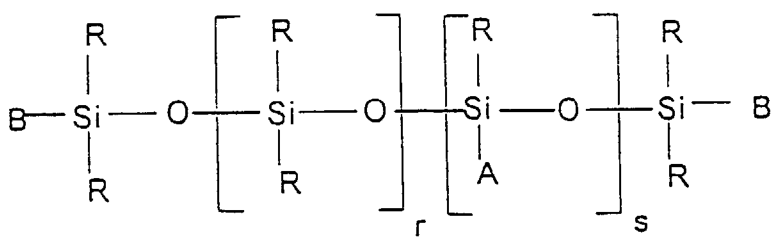

(1)

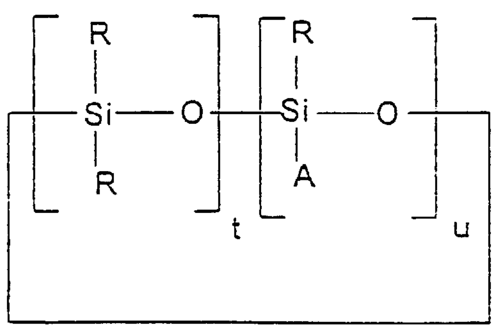

(2)

in which:

- R, which may be identical or different, are chosen from saturated or unsaturated, linear or branched $C_1$-$C_{10}$ alkyl radicals, the phenyl radical and the 3,3,3-trifluoropropyl radical, at least 80%, in numerical terms, of the radicals R being the methyl radical,
- B, which may be identical or different, are chosen from radicals R and the radical A,
- r is an integer chosen between 0 and 3 inclusive,
- s is 0 or 1 and if s is 0, at least one of the two symbols B is A,

- u is equal to 1 or 2,
- t is an integer between 0 and 5 inclusive,
- t + u is greater than or equal to 3,

and A is a radical of formula (3) below:

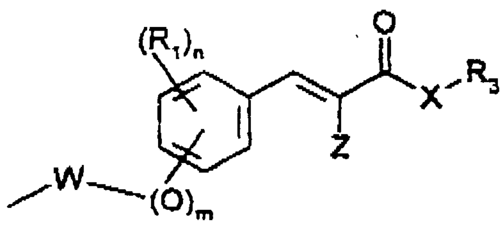

(3)

in which:

- $R_1$ represents a linear or branched $C_1$-$C_{10}$ alkoxy radical, a linear or branched $C_1$-$C_{10}$ alkyl radical, a linear or branched $C_2$-$C_8$ alkenyl radical or a radical $-OSi(CH_3)_3$, it being possible for two adjacent groups $R_1$ to form, together, an alkylidenedioxy group in which the alkylidene group contains 1 or 2 carbon atoms,
- n is 0, 1 or 2,
- m is 0 or 1,
- Z is hydrogen or a radical $-(C=O)YR_3$,
- X and Y, which may be identical or different, independently represent oxygen or a radical $-NR_2-$
- W is a saturated or unsaturated, linear or branched $C_1$-$C_6$ alkanediyl radical, optionally substituted with a hydroxyl or saturated or unsaturated, linear or branched $C_2$-$C_8$ alkyl radical,
- $R_2$ is, independently, hydrogen or a $C_1$-$C_8$ alkyl radical,
- $R_3$ is, independently, a linear or branched $C_1$-$C_{12}$ alkyl radical,
- it being possible for $R_2$ and $R_3$, taken together, to form a heterocycle,

with the condition that when Z = H, then X is the radical $-NR_2-$.

**2.** Compound according to Claim 1, characterized in that it has at least one of the following characteristics:

- R is methyl,
- B is methyl,
- r is 0 or 1,
- s is 1,
- t + u is between 3 and 5,
- n is 0 or 1,
- $R_2$ is methoxy,
- $R_3$ is methyl or ethyl,
- m is 1.

**3.** Compound according to Claim 1 or 2, characterized in that it has all of the following characteristics:

- R is methyl,
- B is methyl,
- r = 0,
- s = 1,
- n = 0,
- m = 1.

**4.** Compound according to any one of Claims 1 to 3, characterized in that it is chosen from:

- diethyl 4-[2-methyl-3-[1,3,3,3-tetramethyl-1-[(trimethylsilyl)oxy]disiloxanyl]propyloxy]benzylidenemalonate,
- diethyl 3-[2-methyl-3-[1,3,3,3-tetramethyl-1-[(trimethylsilyl)oxy]disiloxanyl]propyloxy]benzylidenemalonate,
- ethyl 3-[4-[2-methyl-3-[1,3,3,3-tetramethyl-1-[(trimethylsilyl)oxy]disiloxanyl]propyloxy]phenyl]-2-(piperidine-1-carbonyl)acrylate,
- diethyl 4-[3-[1,3,3,3-tetramethyl-1-[(trimethylsilyl)oxy]disiloxanyl]propyloxy]benzylidenemalonate,
- dimethyl 4-[3-[1,3,3,3-tetramethyl-1-[(trimethylsilyl)oxy]disiloxanyl]propyloxy]benzylidenemalonate,
- diethyl 4-[3-[1,3,3,3-tetramethyl-1-[(trimethylsilyl)oxy]disiloxanyl]-2-propenyloxy]benzylidenemalonate,
- diethyl 4-[3-[1,3,3,3-tetramethyl-1-(trimethyisilyl)oxy]disiloxanyl]-2-vinylidene-ethanoxy]benzylidenemalonate.

**5.** Process for the preparation of a compound as defined in any one of Claims 1 to 4, characterized in that it comprises the following step:

- hydrosilylation of a compound of formula (6) below:

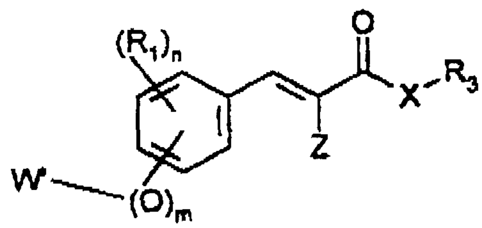

(6)

in which $R_1$, $R_3$, X, Z and n have the same meaning as in formula (3) of Claim 1 and in which W' has the same meaning as W in formula (3) of Claim 1 but ends either with a double bond or with a triple bond, is carried out, in the presence of a catalytically effective amount of a platinum catalyst,

with a derivative of formula (4) or (5) below:

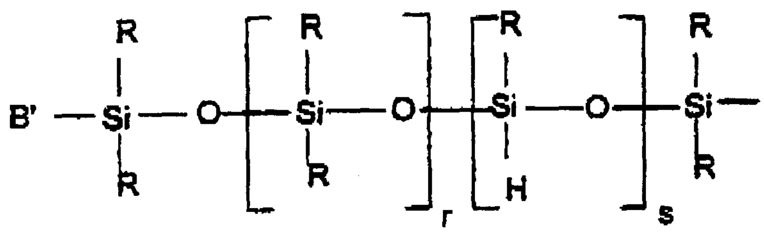

(4)

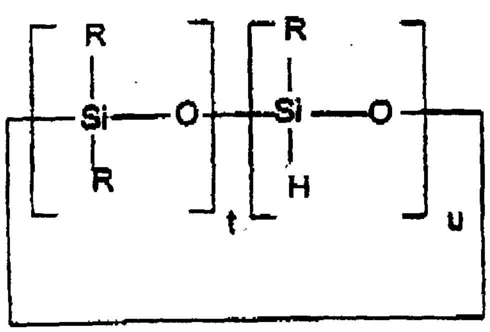

(5)

in which:

- R, r, s, t and u have the meaning given in Claim 1 for formulae (1) and (2),

- B', which may be identical or different, are chosen from the radicals R and a hydrogen atom.

**6.** Process for the preparation of a compound of formula (1) or (2), characterized in that it comprises the following steps:

i) a benzaldehyde of formula (7) below:

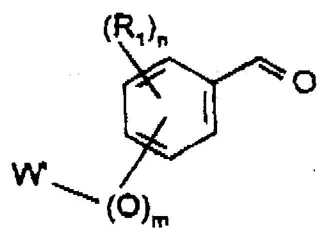

(7)

in which W', R1, m and n have the same meaning as in formula (6) of Claim 5, is reacted with a compound of formula (4) or (5) below:

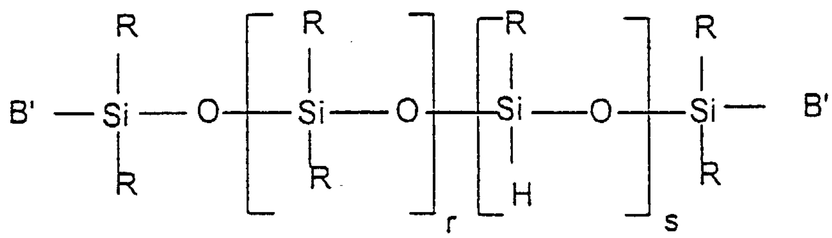

(4)

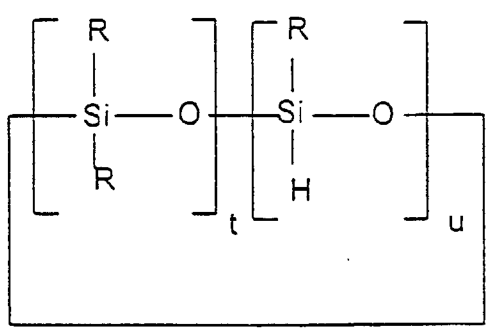

(5) ,

in which:

- R, r, s, t and u have the meaning given above for formulae (1) and (2),
- B', which may be identical or different, are chosen from the radicals R and a hydrogen atom,

in order to obtain the corresponding silicone benzaldehyde,
ii) an organic derivative chosen from those of formula (8) below:

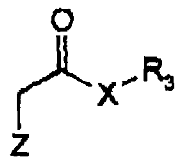

(8)

in which Z, X and $R_3$ have the same meaning as in formula (6) of Claim 5, is reacted with the silicone benzaldehyde obtained in step i).

**7.** Plastic composition, characterized in that it comprises at least one compound as defined in any one of Claims 1 to 4.

**8.** Cosmetic composition intended to protect the skin and/or the hair against UV radiation, comprising, in a cosmetically acceptable support, at least one compound as defined in any one of Claims 1 to 4.

**9.** Composition according to Claim 8, characterized in that the compound as defined in any one of Claims 1 to 4 is present in the composition in a proportion ranging from 0.1 to 20% by weight, relative to the total weight of the composition, preferably ranging from 0.5% to 10% by weight, relative to the total weight of the composition.

**10.** Use of at least one compound as defined in any one of Claims 1 to 4 for the manufacture of compositions intended to protect sensitive material against ultraviolet radiation, in particular solar radiation.

**11.** Use of at least one compound as defined in any one of Claims 1 to 4, as an agent for screening out UV radiation, for the manufacture of cosmetic compositions intended to protect the skin and/or the hair against UV radiation.

**12.** Use of at least one compound as defined in any one of Claims 1 to 4 as an agent for screening out UV radiation, for the preparation of a medicinal product intended to prevent the harmful effects of UV radiation.

**13.** Use of a compound as defined in any one of Claims 1 to 4 as an agent for controlling the colour of the skin, for the manufacture of cosmetic compositions.